(19) **Europäisches Patentamt / European Patent Office / Office européen des brevets**

(11) **EP 3 856 040 B1**

(12) **EUROPEAN PATENT SPECIFICATION**

(45) Date of publication and mention
of the grant of the patent:
**03.01.2024 Bulletin 2024/01**

(21) Application number: **18808517.9**

(22) Date of filing: **22.11.2018**

(51) International Patent Classification (IPC):
*A61B 8/10* (2006.01)    *A61B 3/16* (2006.01)
*B05B 17/06* (2006.01)    *B06B 3/02* (2006.01)
*B23K 20/10* (2006.01)

(52) Cooperative Patent Classification (CPC):
**A61B 8/10; A61B 3/165; B05B 17/0623;** B06B 3/02

(86) International application number:
**PCT/IB2018/059214**

(87) International publication number:
**WO 2020/065387 (02.04.2020 Gazette 2020/14)**

(54) **ULTRASONIC TONOMETER AND ULTRASONIC ACTUATOR**

ULTRASCHALLTONOMETER UND ULTRASCHALLAKTUATOR

TONOMÈTRE ULTRASONORE ET ACTIONNEUR ULTRASONORE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **28.09.2018 ES 201830940**

(43) Date of publication of application:
**04.08.2021 Bulletin 2021/31**

(73) Proprietor: **Nidek Co., Ltd.
Gamagori-shi, Aichi 443-0038 (JP)**

(72) Inventors:
  • **MIWA, Tetsuyuki
    Gamagori-shi, Aichi 443-0038 (JP)**
  • **UEMURA, Tsutomu
    Gamagori-shi, Aichi 443-0038 (JP)**

  • **CARDONI, Andrea
    28020 Madrid (ES)**
  • **MARTÍNEZ GONZÁLEZ, Ignacio
    28821 Madrid (ES)**

(74) Representative: **Hoefer & Partner Patentanwälte
mbB
Pilgersheimer Straße 20
81543 München (DE)**

(56) References cited:
**EP-A1- 2 459 268        EP-B1- 2 459 268
DE-U1-202012 010 508    JP-A- S6 311 147
JP-A- S63 302 842        US-A- 5 469 848
US-A1- 2006 090 956**

EP 3 856 040 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

[Technical Field]

The present disclosure relates to an ultrasonic actuator that emits an ultrasonic wave and an ultrasonic tonometer that measures an intraocular pressure of a subject's eye using an ultrasonic wave.

[Background Art]

As a non-contact tonometer, an air injection type tonometer is still common. The air injection type tonometer converts an air pressure in a predetermined deformed state into an intraocular pressure by detecting a deformed state of a cornea when air is injected into the cornea and an air pressure of the air injected into the cornea.

As a non-contact tonometer, an ultrasonic tonometer for measuring the intraocular pressure using an ultrasonic wave is proposed (See Patent Document 1). The ultrasonic tonometer of Patent Literature 1 converts a radiation pressure in a predetermined deformed state into an intraocular pressure by detecting a deformed state of a cornea when the cornea is irradiated with the ultrasonic wave and a radiation pressure of the ultrasonic wave radiated to the cornea.

As an ultrasonic tonometer, an apparatus that measures the intraocular pressure based on a relationship between characteristics (amplitude, phase) of a reflected wave from a cornea and the intraocular pressure is proposed (See Patent Document 2).

EP 2 459 268 A1 describes a sonotrode and its use for transdermal delivery of therapeutic or cosmetic compounds. The sonotrode comprises a tubular neck attached at a proximal end to a solid head and ending at a distal end in a flared foot. [Prior Art Document]

[Patent Document]

[Patent Document 1] JP-A-H05-253190

[Patent Document 2] JP-A-2009-268651

[Summary of Invention]

[Problems to be Solved by Invention]

However, in the ultrasonic tonometer as a related-art, the ultrasonic wave cannot be properly irradiated to a cornea of a subject's eye. For example, in the tonometer of Patent Document 1, the ultrasonic wave cannot be properly irradiated to the cornea, and the cornea cannot be actually flattened or depressed. For example, in the tonometer of Patent Document 2, the ultrasonic wave cannot be properly irradiated to the cornea, and the characteristics of the reflected wave cannot be sufficiently detected.

In view of the problem of the related-art, an object of the present disclosure is to provide an ultrasonic actuator and an ultrasonic tonometer that are capable of properly irradiating a subject's eye with an ultrasonic wave.

[Means for Solving Problems]

This object is solved by the features of the independent claim. The dependent claims contain advantageous embodiments of the present invention.

[Brief Description of Drawings]

FIG. 1 is an external view of an ultrasonic tonometer.
FIG. 2 is a schematic view showing an inside of a housing.
FIG. 3 is a schematic cross-sectional view showing a configuration of an ultrasonic actuator.
FIG. 4 is an enlarged cross-sectional view of a part of the ultrasonic actuator.
FIGs. 5A and 5B illustrate a cylindrical cross-section of a sonotrode.
FIG. 6 is a schematic cross-sectional view showing a configuration of the ultrasonic actuator.
FIGs. 7A and 7B illustrate an uneven portion.
FIG. 8 illustrates a part of the sonotrode.
FIG. 9 illustrates a part of the sonotrode.
FIG. 10 is a block diagram illustrating a control system.

<First Example>

Hereinafter, a first example will be described. An ultrasonic tonometer of the first example measures an intraocular pressure of a subject eye using an ultrasonic wave. The ultrasonic tonometer includes an ultrasonic actuator (for example, an ultrasonic actuator 100), for example. The ultrasonic actuator irradiates the subject eye with the ultrasonic wave. The ultrasonic actuator includes an ultrasonic element (for example, an ultrasonic element 110) and a sonotrode (for example, a sonotrode 131), for example. The ultrasonic element generates an ultrasonic wave. The sonotrode propagates the ultrasonic wave generated from the ultrasonic element. The sonotrode includes an uneven portion (for example, an uneven portion 180). The uneven portion is configured with a portion where a thickness of the sonotrode varies in a sound axial direction of the ultrasonic wave (a traveling direction of the ultrasonic wave, a vibration direction of the ultrasonic element, a radiation direction of the ultrasonic wave, a front-back direction of the ultrasonic tonometer). Thus, since the sonotrode includes the uneven portion, the ultrasonic tonometer of the present example can amplify an amplitude of the ultrasonic wave and emit the ultrasonic wave more efficiently.

The sonotrode includes an opening (for example, an opening 101). The opening opens in the sound axial direction of the ultrasonic wave, for example. In this case, the uneven portion may be provided on at least one of an outer surface and an inner surface of the sonotrode. The outer surface of the sonotrode is, for example, a side surface of the sonotrode when an ultrasonic output surface (a subject eye side) of the sonotrode is a front surface. The inner surface of the sonotrode is an inner wall surface of the sonotrode inside the opening. That is, the sonotrode is formed in a hollow cylindrical shape, and the uneven portion may be provided on both an outer peripheral wall surface and an inner peripheral wall surface of the sonotrode, or may be provided on either wall surface. An optical axis of an observation optical system for observing the subject eye or an optical axis of a measurement optical system for measuring the subject eye may be disposed in the opening, for example. Therefore, observation or measurement of the subject eye may be performed through the opening. That is, the ultrasonic tonometer of the present example may include an optical system in which an optical axis is disposed in the opening.

The uneven portion may include a thick portion (for example, a thick portion 181) and a thin portion (for example, a thin portion 182). The thin portion has a thinner thickness of the sonotrode than the thick portion. The thick portion and the thin portion are alternately formed along the sound axis direction. Therefore, unevenness is formed by a difference in thickness between the thick portion and the thin portion. A curvature portion (for example, a curvature portion 183) may be provided between the thick portion and the thin portion. The curvature portion is formed by a curved surface, for example. Thus, a continuous surface is formed between the thick portion and the thin portion, and the ultrasonic wave can be efficiently propagated from the thick portion to the thin portion.

A length of a pair of the thick portion and the thin portion forming the uneven portion may be equal to an integral multiple of a half wavelength of the ultrasonic wave generated from the ultrasonic element. Accordingly, the ultrasonic actuator easily resonates, and the ultrasonic wave can propagate more efficiently.

A plurality of pairs of thick portion and thin portion forming the uneven portion may be provided at intervals of an integral multiple of a half wavelength of the ultrasonic wave. Thus, a vibration mode of the ultrasonic actuator approaches a single mode (primary vibration mode, single vibration mode), and a sound pressure is efficiently increased.

<First Embodiment>

An ultrasonic tonometer of a first embodiment measures the intraocular pressure of the subject eye using the ultrasonic wave, as is the same with the first example. The ultrasonic tonometer (for example, an ultrasonic tonometer 1) of the first embodiment includes an ultrasonic actuator (for example, then ultrasonic actuator 100). The ultrasonic actuator irradiates the subject eye with the ultrasonic wave. The ultrasonic actuator includes an ultrasonic element (for example, the ultrasonic element 110) and a sonotrode (for example, the sonotrode 131).

The ultrasonic element generates an ultrasonic wave. The sonotrode propagates the ultrasonic wave generated from the ultrasonic element. The sonotrode includes an irradiation surface (for example, an irradiation surface 184) and an opening (for example, the opening 101). The irradiation surface is, for example, a surface facing the subject eye. For example, the ultrasonic wave generated from the ultrasonic element propagates through the sonotrode and is output from the irradiation surface into the air. The opening opens in the sound axial direction of the ultrasonic wave, for example. The sonotrode is formed to have a substantially same propagation distance until the ultrasonic wave generated from the ultrasonic element reaches the irradiation surface between an outer surface and an inner surface of the sonotrode. Thus, a wavefront of the ultrasonic wave on the irradiation surface is aligned, and the wavefront of the ultrasonic wave is incident parallel to the irradiation surface. Therefore, the vibration mode of the ultrasonic actuator becomes the single mode, and the ultrasonic wave output from the irradiation surface propagates efficiently in the air. The propagation distance may not exactly coincide between the outer surface and the inner surface of the sonotrode.

The sonotrode includes a propagation distance adjustment portion (for example, an inner groove 185, an outer groove 186). The propagation distance adjustment portion is provided to reduce a difference of the propagation distance between the outer surface and the inner surface of the sonotrode. For example, the propagation distance adjustment portion is provided so that the propagation distance coincides between the outer surface and the inner surface of the sonotrode. Thus, the wavefront of the ultrasonic wave on the irradiation surface is aligned. The propagation distance adjustment portion may be provided on both the outer surface and the inner surface of the sonotrode, or may be provided only on the inner surface.

According to the invention, the propagation distance adjustment portion is a groove having a curved surface. As the ultrasonic wave propagates along the groove, the propagation distance of the ultrasonic wave can be increased. Thus, the propagation distance of the ultrasonic wave is adjusted, and the wavefront of the ultrasonic wave on the irradiation surface is aligned.

The irradiation surface is inclined to a side of the ultrasonic element and toward a center of the sound axis. That is, the irradiation surface is inclined to the side of the ultrasonic element and toward a center of the opening. Thus, the ultrasonic wave can be converged toward the subject eye. The irradiation surface may have a curvature. For example, the irradiation surface may be an inclined curved surface.

The ultrasonic actuators in the first example and first embodiment may be Langevin type. The Langevin type ultrasonic actuator has a shape in which an ultrasonic element is sandwiched by mass members, for example. Accordingly, the Langevin type ultrasonic actuator can obtain high output.

The ultrasonic actuators in the first example and first embodiments may be used not only in the tonometer but also in other fields. For example, the ultrasonic actuator may be used in a medical equipment of dermatology or the like other than ophthalmology. The ultrasonic actuator may be used in a device utilizing a high-power ultrasonic wave.

<Example>

An example according to the present disclosure will be described below. An ultrasonic tonometer of the present example measures the intraocular pressure of the subject eye in a non-contact manner using an ultrasonic wave, for example. The ultrasonic tonometer measures the intraocular pressure by optically or acoustically detecting a shape change or vibration of the subject eye, for example, when the subject eye is irradiated with the ultrasonic wave. For example, the ultrasonic tonometer continuously irradiates a cornea with a pulse wave or a burst wave, and calculates the intraocular pressure based on output information of the ultrasonic wave when the cornea is deformed into a predetermined shape. The output information is, for example, an ultrasonic sound pressure, an acoustic radiation pressure, an irradiation time (for example, an elapsed time after a trigger signal is input), or a frequency. When the cornea of the subject eye is deformed, the ultrasonic sound pressure, the acoustic radiation pressure, or an acoustic flow is used, for example.

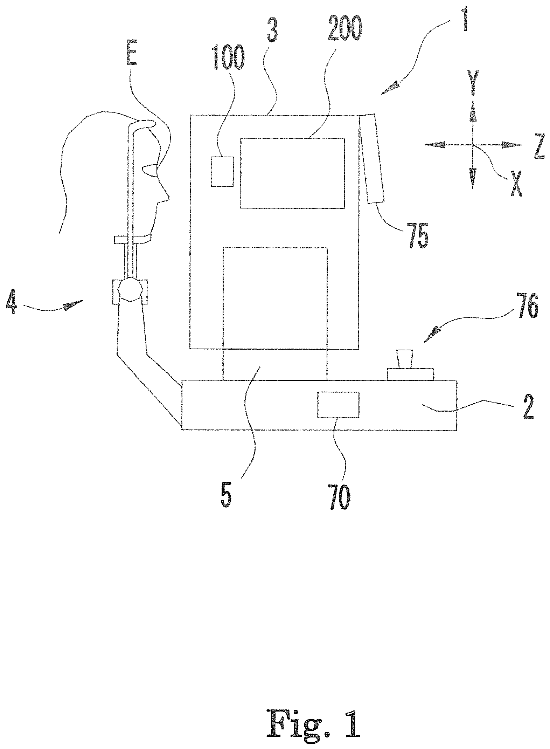

FIG. 1 shows an appearance of the ultrasonic tonometer. The ultrasonic tonometer 1 includes a base 2, a housing 3, a face support unit 4, a drive unit 5, and the like, for example. An ultrasonic actuator 100 described later, an optical unit 200, and the like are arranged inside the housing 3. The face support unit 4 supports a face of a subject. The face support unit 4 is installed on the base 2, for example. The drive unit 5 moves the housing 3 with respect to the base 2 for alignment, for example.

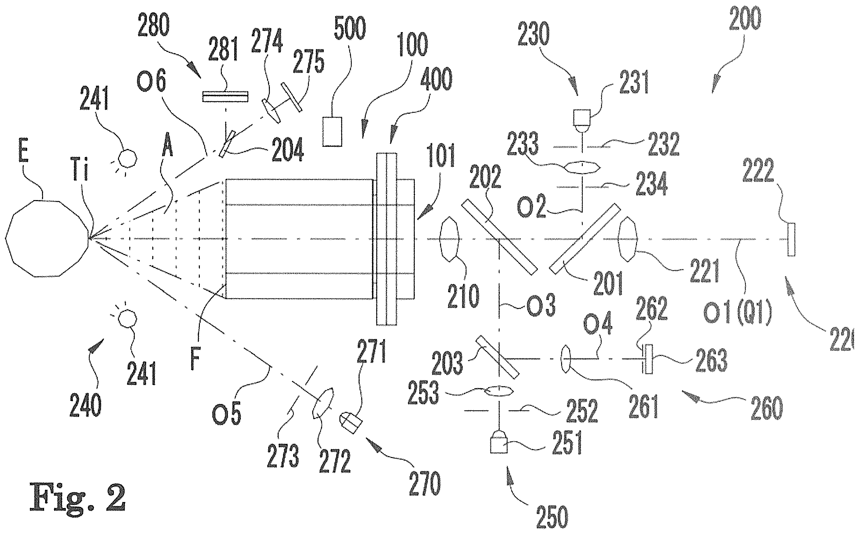

FIG. 2 is a schematic view of a main configuration inside the housing.

An ultrasonic actuator 100 and the optical unit 200 are arranged inside the housing 3, for example. The ultrasonic actuator 100 and the optical unit 200 will be described in order using FIG. 2.

The ultrasonic actuator 100 irradiates a subject eye E with an ultrasonic wave, for example. For example, the ultrasonic actuator 100 irradiates a cornea with the ultrasonic wave to generate an acoustic radiation pressure on the cornea. The acoustic radiation pressure is, for example, a force acting in a direction that a sound wave travels. The ultrasonic tonometer 1 of the present example deforms the cornea using the acoustic radiation pressure, for example. The ultrasonic unit of the example has a cylindrical shape, and an optical axis O1 of the optical unit 200 described later is disposed in the opening 101 at the center.

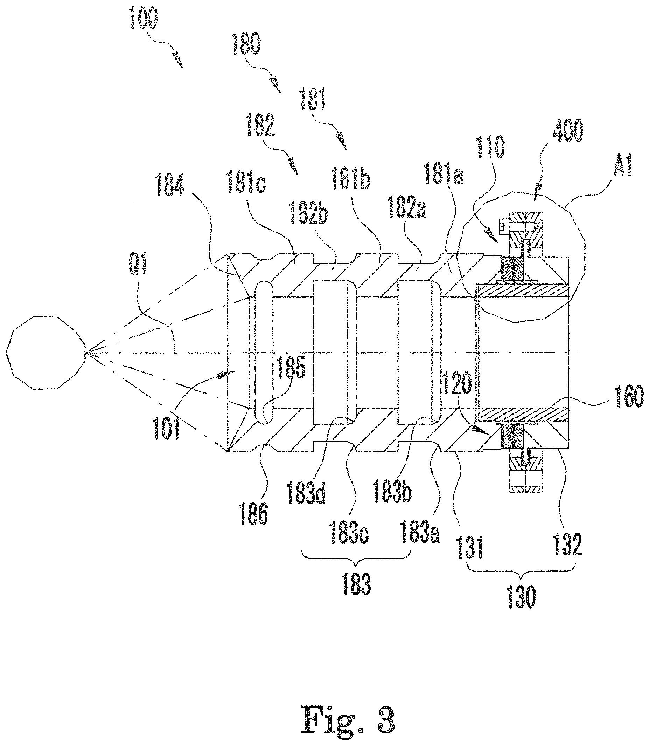

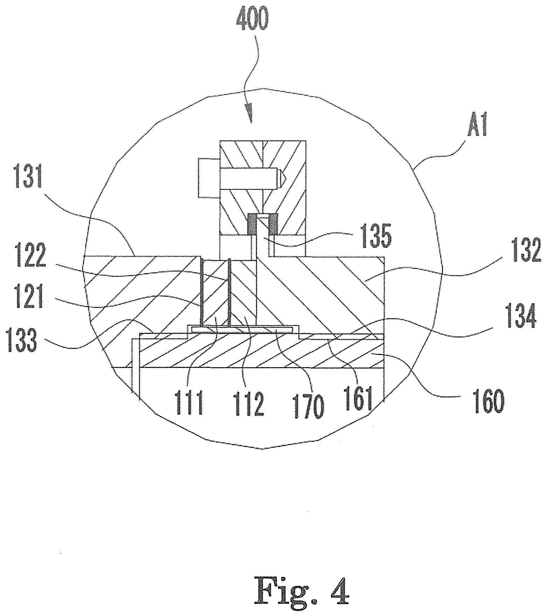

The ultrasonic actuator 100 of the present example is a so-called Langevin type vibrator. As shown in FIG. 3, the ultrasonic actuator 100 includes an ultrasonic element 110, an electrode 120, a mass member 130, and a fastening member 160, for example. The ultrasonic element 110 generates an ultrasonic wave. The ultrasonic element 110 may be a voltage element (for example, a piezoelectric ceramic) or a magnetostrictive element. The ultrasonic element 110 of the example has a ring shape. For example, the ultrasonic element 110 may be a laminate of a plurality of piezoelectric elements. FIG. 4 is an enlarged view of a region A1 in FIG. 3. In the example, as shown in FIG. 4, two laminated piezoelectric elements (for example, the piezoelectric element 111 and the piezoelectric element 112) are used as the ultrasonic element 110. For example, the electrodes 120 (electrode 121, electrode 122) are connected to the two piezoelectric elements, respectively. The electrode 121 and the electrode 122 of the example are in a ring shape, for example.

The mass member 130 sandwiches the ultrasonic element 110, for example. Since the mass member 130 sandwiches the ultrasonic element 110, the mass member 130 increases a tensile strength of the ultrasonic element 110, and the ultrasonic element 110 can withstand a strong vibration, for example. Therefore, a high-power ultrasonic wave can be generated. The mass member 130 may be a metal block, for example. For example, the mass member 130 includes a sonotrode (which is also referred to as a horn or a front mass) 131 and a back mass 132.

The sonotrode 131 is a mass member disposed in front (at a subject eye side) of the ultrasonic element 110. The sonotrode 131 propagates and amplifies the ultrasonic wave generated from the ultrasonic element 110. The sonotrode 131 of the example has a hollow cylindrical shape (hollow tubular shape). A female screw portion 133 is formed on a part of an inner circle side of the sonotrode 131. The female screw portion 133 is screwed to a male screw portion 161 formed in a fastening member 160 described later.

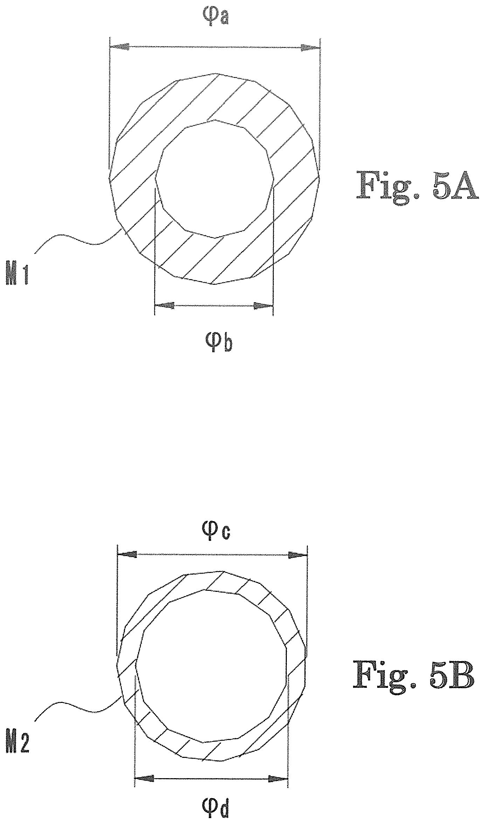

The sonotrode 131 of the example is a hollow cylinder having an uneven thickness. For example, the sonotrode 131 has a shape that an outer diameter and an inner diameter vary with respect to the sound axis O1 direction (longitudinal direction) of the hollow cylinder. For example, as shown in FIG. 3, the uneven portion 180 including the thick portion 181 and the thin portion 182 is provided. FIG. 5A shows a cross section when the thick portion 181 is cut perpendicular to the sound axis direction. FIG. 5B shows a cross section when the thin portion 182 is cut perpendicular to the sound axis direction. The thick portion 181 has an outer diameter φa and an inner diameter φb. The thin portion 182 has an outer diameter φc and an inner diameter φd. The outer diameter φa of the thick portion 181 is larger than the outer diameter φc of the thin portion 182, and the inner diameter φb of the thick portion 181 is smaller than the inner diameter φd of the thin portion 182. A cross-sectional area M1 of the hollow cylinder of the thick portion 181 is larger than a cross-sectional area M2 of the hollow cylinder of the thin portion 182.

The thick portion 181 and the thin portion 182 amplify the ultrasonic wave generated from the ultrasonic element 110. For example, the ultrasonic wave is amplified when the ultrasonic wave propagates from the thick portion 181 to the thin portion 182. This is due to the horn effect. For example, when water at a constant flow rate flows from a thick pipe to a thin pipe, a flow velocity in the thin pipe increases. An amplitude gain of the example is given by the area ratio (M1/M2) of the cross-sectional area M1 of the thick portion 181 and the cross-sectional area M2 of the thin portion 182.

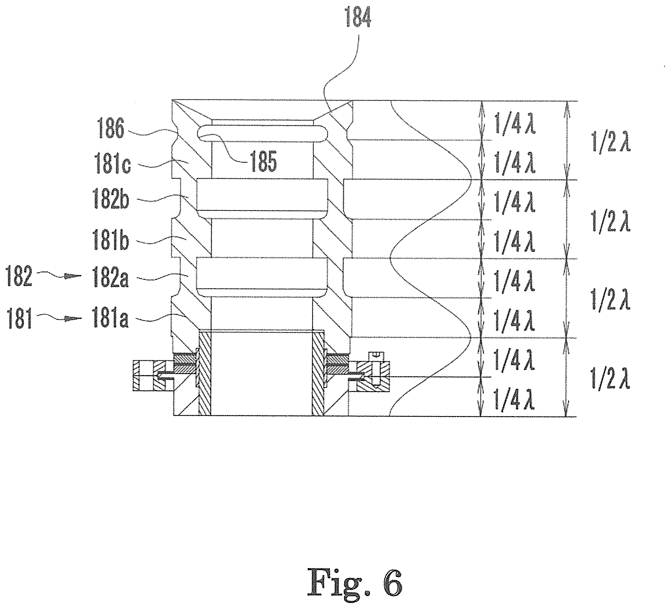

As shown in FIG. 6, pairs of the thick portion 181 and the thin portion 182 forming the uneven portion 180 are provided at an integral multiple of a half wavelength of the ultrasonic wave generated from the ultrasonic element 110 along the sound axis direction from an end surface (the irradiation surface 184 or a rear surface) of the ultrasonic actuator. Accordingly, the ultrasonic actuator 100 easily resonates, and the ultrasonic wave can propagate more efficiently. The thick portion 181 and the thin portion 182 are arranged at an intervals of a quarter wavelength λ of the ultrasonic wave, respectively. For example, as shown in FIG. 6, a length of a thick portion 181a in the sound axis direction is 1/4 λ. A length of a thin portion 182a which is adjacent to a thick portion 181a is 1/4 λ. Similarly, a length of a thick portion 181b, a thin portion 182b, and a thick portion 181c in the sound axis direction is 1/4 λ. Since the thick portion 181 and the thin portion 182 are provided at the intervals of 1/4 λ, the vibration mode of the ultrasonic actuator 100 is likely to be the single mode. Accordingly, the entire ultrasonic actuator 100 vibrates efficiently, and a high sound pressure can be generated.

A plurality of the thick portions 181 and the thin portions 182 of this example are provided at intervals of 1/4 wavelength. That is, the uneven portion 180 is provided with a plurality of pairs of the thick portion 181 and the thin portion 182 at half wavelength intervals. In this case, the ultrasonic wave generated by the ultrasonic element 110 propagates from the thick portion 181a to the thin portion 182a, and then alternately propagates the thick portion 181 and the thin portion 182 in the order of the thick portion 181b, the thin portion 182b, and the thick portion 181c. Since the plurality of the thick portions 181 and the thin portions 182 are providing in this manner, the vibration mode approaches the single mode, and the sound pressure can be efficiently increased. Further, the ultrasonic wave is repeatedly amplified by the plurality of the thick portions 181 and the plurality of thin portions 182, the sound pressure can be further increased.

In the plurality of the thick portions 181 (for example, the thick portion 181a, the thick portion 181b, the thick portion 181c) each thick portion may have the same inner and outer diameters with the other thick portion, or may have different inner and outer diameters from the other thick portion. Similarly, in the plurality of the thin portions 182 (for example, the thin portion 182a, the thin portion 182b), each thin portion may have the same inner and outer diameters with the other thin portion, or may have different inner and outer diameters from the other thin portion.

A curvature portion 183 is provided at a portion where the ultrasonic wave enters from the thick portion 181 having the large cross-sectional area M1 to the thin portion 182 having the small cross-sectional area M2. The curvature part 183 is, for example, a curved surface (a shape having a curvature). In the example of FIG. 3, a curvature portion 183a and a curvature portion 183b are provided between the thick portion 181a and the thin portion 182a. The curvature portion 183a is provided on the outer surface of the sonotrode 131, and the curvature portion 183b is provided on the inner surface of the sonotrode 131. Similarly, a curvature portion 183c and a curvature portion 183d are provided between the thick portion 181b and the thin portion 182b. The curvature part 183c is provided on the outer surface of the sonotrode 131, and the curvature part 183d is provided on the inner surface of the sonotrode 131. For example, the curvature portion 183 is formed by a curved surface such that a variation in diameter between the thick portion 181 and the thin portion 182 is continuous.

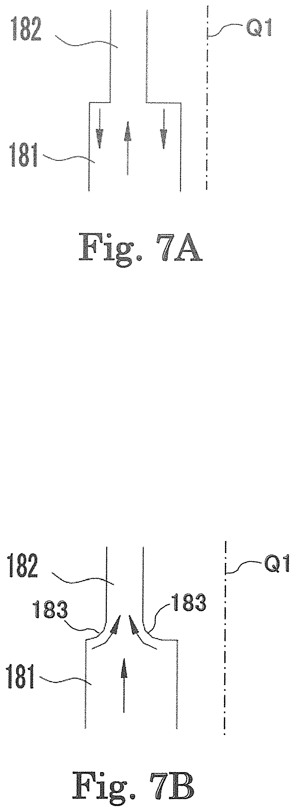

If there is no curvature portion 183 between the thick portion 181 and the thin portion 182 as shown in FIG. 7A, the ultrasonic wave transmitted in the direction of the sound axis Q1 through the thick portion 181 are reflected by the thin portion 182 in a direction opposite to the traveling direction. However, as in the present example of FIG. 7B, when the curvature portion 183 is provided between the thick portion 181 and the thin portion 182, the ultrasonic wave transmitted in the direction of the sound axis Q1 through the thick portion 181 is suppressed from being reflected by the thin portion 182, and the ultrasonic wave can be more efficiently propagated to the thin portion 182.

As described above, since the uneven portion such as the thick portion 181 and the thin portion 182 is provided with the sonotrode, the amplitude of the ultrasonic wave propagated from the thick portion 181 to the thin portion 182 is amplified, and the ultrasonic wave is more efficiently propagated from the irradiation surface 184 into the air. Thus, it is possible to irradiate the subject eye with the ultrasonic wave having an output sufficient to measure the intraocular pressure. Further, since the curvature portion 183 provides a curvature between the thick portion 181 and the thin portion 182, the ultrasonic wave can be smoothly propagated from the thick portion 181 to the thin portion 182.

The sonotrode 131 of the present example has a shape that converges the ultrasonic wave. According to the invention, the irradiation surface (an end surface on the subject eye side) 184 of the sonotrode 131 is inclined to a side of the ultrasonic element 110 and toward the center (the sound axis Q1) of the opening 101. For example, the irradiation surface 184 has a tapered shape. The irradiation surface 184 may be an inclined surface having a curvature. The irradiation surface 184 may have a spherical shape whose radius is a working distance of the ultrasonic actuator 100. Since the irradiation surface 184 is inclined, the ultrasonic wave emitted from the irradiation surface 184 converges to a target position, and a large sound pressure is generated.

Since the irradiation surface 184 of the sonotrode 131 is inclined, a time until the ultrasonic wave from the ultrasonic element 110 reaches the irradiation surface 184 is different between the outer surface (outer peripheral side) and the inner surface (inner peripheral side) of the sonotrode 131. For example, since a propagation path of the ultrasonic wave propagating through the outer surface of the sonotrode 131 is longer than a propagation path of the ultrasonic wave propagating through the inner surface, the ultrasonic wave propagating through the outer surface reaches the irradiation surface 184 later than the ultrasonic wave propagating through the inner surface. Therefore, the wavefront of the ultrasonic wave shifts between the outer side and the inner side of the irradiation surface 184. In this case, since a complicated vibration mode in the irradiation surface 184 occurs, it is difficult for the ultrasonic wave to propagate into the air.

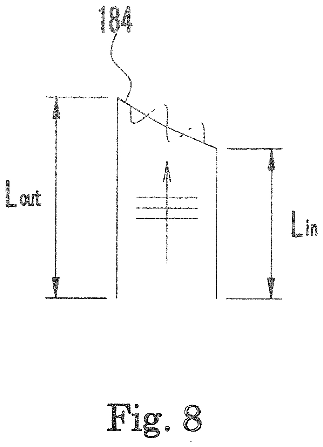

FIG. 8 shows the wavefront of the ultrasonic wave incident on the irradiation surface 184. When the wavefront of the ultrasonic wave propagates perpendicularly to the ultrasonic element 110 and enters the irradiation surface 184 as it is, the wavefront of the ultrasonic wave is obliquely incident on the irradiation surface 184. In this case, since a time difference occurs between the inner surface side and the outer surface side until the same wavefront of the ultrasonic wave reaches the irradiation surface 184, the displacement on the irradiation surface 184 by the ultrasonic wave is different according to a position in the irradiation surface 184. Thus, since the irradiation surface 184 is not sufficiently displaced, the ultrasonic wave is not efficiently emitted.

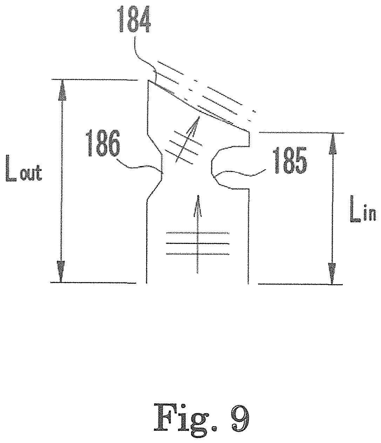

Therefore, as shown in FIG. 9, the sonotrode 131 of the example includes an inner groove 185 and an outer groove 186. The inner groove 185 is a groove formed inside the opening 101 of the sonotrode 131. The outer groove 186 is a groove formed outside the sonotrode 131. A creepage distance of the inner groove 185 is different from a creepage distance of the outer groove 186. According to the invention, the creepage distance is a distance in the direction of the sound axis Q1 along the outer surface or the inner surface of the sonotrode 131. For example, since the inner groove 185 is cut deeper in the thickness direction than the outer groove 186, the creepage distance of the inner groove 185 is longer than the creepage distance of the outer groove 186. The difference in the creepage distance between the inner groove 185 and the outer groove 186 is used to make the wavefront of the ultrasonic wave parallel to the irradiation surface 184.

The condition in which the wavefront of the ultrasonic wave is parallel to the irradiation surface 184 is shown in equation (1).

$$\mathrm{Lin} = \mathrm{Lout} \quad ...(1)$$

That is, when an inner creepage distance Lin and an outer creepage distance Lout are equal, the wavefront of the ultrasonic wave is parallel to the irradiation surface 184. In order to meet with the equation (1), for example, the depth of each groove of the inner groove 185 and the outer groove 186 are set so as to cancel the difference of the creepage distance between the inner surface and the outer surface caused by the inclination of the irradiation surface 184. Accordingly, since the vibration mode of the irradiation surface 184 becomes a single mode, the ultrasonic wave can be efficiently propagated into the air.

As described above, since the ultrasonic tonometer 1 of the present example includes the inner groove 185 and the outer groove 186 having a different creepage distance from each other, the wavefront of the ultrasonic wave can be made parallel to the irradiation surface 184 even though the irradiation surface 184 is inclined. As a result, the ultrasonic wave is converged on the subject eye, and the vibration mode of the irradiation surface 184 becomes the single-mode. Therefore, a propagation efficiency or emission efficiency of the ultrasonic wave with respect to the air is improved, and a sound pressure (or acoustic radiation pressure) capable of sufficiently deforming the cornea can be generated.

A back mass 132 is a mass member disposed behind the ultrasonic element 110. The back mass 132 sandwiches the ultrasonic element 110 together with the sonotrode 131. As a result, the back mass 132 couples the ultrasonic element 110 and the sonotrode 131. The back mass 132 has a cylindrical shape, for example. A female screw portion 134 is formed in a part of an inner circular portion of the back mass 132. The female screw portion 134 is screwed to a male screw portion 161 of the fastening member 160 described later. The back mass 132 includes a flange portion 135. The flange portion 135 is held by a mount unit 400.

The fastening member 160 fastens the mass member 130 and the ultrasonic element 110 sandwiched by the mass member 130, for example. The fastening member 160 is a hollow bolt, for example. The fastening member 160 is, for example, in a cylindrical shape and includes the male screw portion 161 in an outer circular portion. The male screw portion 161 of the fastening member 160 is screwed to the female screw portions 133, 134 formed inside the sonotrode 131 and inside the back mass 132. The sonotrode 131 and the back mass 132 are tightened in a direction of pulling each other by the fastening member 160. As a result, the ultrasonic element 110 sandwiched between the sonotrode 131 and the back mass 132 is tightened and a pressure is applied to the ultrasonic element 110.

The ultrasonic actuator 100 may include an insulating member 170. The insulating member 170 prevents, for example, the electrode 120 or the ultrasonic element 110 from contacting with the fastening member 160. The insulating member 170 is disposed between the electrode 120 and the fastening member 160, for example. The insulating member 170 is, for example, in a sleeve shape.

As shown in FIG. 6, the entire structure of the ultrasonic actuator 100 including the thick portion 181, the thin portion 182, and the back mass 132 of the sonotrode 131 is provided with a length based on a half of the wavelength λ of the ultrasonic wave generated from the ultrasonic element 110. For example, the total length of the ultrasonic actuator 100 is set to an integral multiple of 1/2 λ. This is because the vibration of the half wavelength resonance in which a vibration amplitude is large at both ends of the ultrasonic actuator 100 is generated in the sound axis Q1 direction. In this way, the entire ultrasonic actuator 100 vibrates efficiently and generates a high sound pressure by making the shape with reference to 1/2 λ. Thus, the ultrasonic actuator 100 can irradiate the subject eye with an ultrasonic wave having an output sufficient to deform a cornea into a predetermined shape.

Each length of the sonotrode 131, the ultrasonic element 110, and the back mass 132 in the direction of the sound axis Q1 is considered so that the propagation path length due to the sound velocity (speed of waves transmitted through a medium of vibration) or the shape is 1/2 λ.

The sonotrode 131 and the back mass 132 may be formed of different materials each other. For example, the back mass 132 may be formed of a material stiffer than the material of the sonotrode 131. For example, in the present example, titanium, which is a softer material, is used for the sonotrode 131, and steel, which is stiffer than titanium, is used for the back mass 132. Titanium has low acoustic losses therefore increases the overall Q value of the ultrasonic actuator 100. Since different materials are used for the sonotrode 131 and the back mass 132, the Q value of the actuator 100 increases, and the vibrations of each portion are easily synchronized. Therefore, the ultrasonic actuator 100 can output a higher sound pressure. The higher the Q value is, the more the vibration mode is closed to the single mode. Therefore, the ultrasonic wave can be propagated more efficiently.

<Optical Unit>

The optical unit 200 performs observation or measurement of a subject eye, for example (see FIG. 2). The optical unit 200 includes an objective system 210, an illumination optical system 240, an observation system 220, a fixed target projection system 230, an index projection system 250, a deformation detection system 260, a corneal thickness measuring system 270, a Z alignment detection system 280, a dichroic mirror 201, a beam splitter 202, a beam splitter 203, and a beam splitter 204, for example.

The objective system 210 is, for example, an optical system for taking light from the outside of the housing 3 into the optical unit 200 or emitting light from the optical unit 200 outside the housing 3. The objective 210 includes an optical element, for example. The objective system 210 may include an optical element (objective lens, relay lens, or the like).

The illumination optical system 240 illuminates the subject eye. The illumination optical system 240 illuminates the subject eye with infrared light, for example. The illumination optical system 240 includes an illumination light source 241, for example. The illumination light source 241 is disposed obliquely forward of the subject eye, for example. The illumination light source 241 emits infrared light, for example. The illumination optical system 240 may include a plurality of illumination light sources 241.

The observation system 220 captures an observation image of the subject eye, for example. The observation system 220 captures an anterior ocular image of the subject eye, for example. The observation system 220 includes a light receiving lens 221 and a light receiving element 222, for example. The observation system 220 receives light from the illumination light source 241 reflected by the subject eye, for example. The observation system 220 receives a reflected light beam from the subject eye travelling about the optical axis O1, for example. For example, the reflected light from the subject eye passes through the opening 101 of the ultrasonic actuator 100 and is received by the light receiving element 222 via the objective 210 and the light receiving lens 221.

The fixed target projection system 230 projects a fixed target onto the subject eye, for example. The fixed target projection system 230 includes a target light source 231, a diaphragm 232, a light projection lens 233, and a diaphragm 234, for example. Light from the target light source 231 passes through the diaphragm 232, the projection lens 233 and the diaphragm 232 along an optical axis O2, and is reflected by the dichroic mirror 201. The dichroic mirror 201 makes the optical axis O2 of the fixed target projection system 230 coaxial with the optical axis O1, for example. Light from the target light source 231 reflected by the dichroic mirror 201 passes through the objective 210 along the optical axis O1, and is irradiated to the subject eye. Since the target of the fixed target projection system 230 is fixedly seen by a subject, a line of sight of the subject is stabilized.

The index projection system 250 projects an index onto the subject eye, for example. The index projection system 250 projects an index for XY alignment on the subject eye. The index projection system 250 includes an index light source (for example, an infrared light source) 251, a diaphragm 252, and a light projecting lens 253, for example. Light from the index light source 251 passes through the diaphragm 252 and the projection lens 253 along an optical axis O3, and is reflected by the beam splitter 202. The beam splitter 202 makes the optical axis O3 of the index projection system 250 coaxial with the optical axis O1, for example. The light of the index light source 251 reflected by the beam splitter 202 passes through the objective 210 along the optical axis O1, and is irradiated to the subject eye. The light of the index light source 251 irradiated to the subject eye is reflected by the subject eye, passes through the objective 210 and the light receiving lens 221 along the optical axis O1 again, and is received by the light receiving element 222. The index received by the light receiving element 222 is used for the XY alignment, for example. In this case, for example, the index projection system 250 and the observation system 220 function as XY alignment detection means.

The deformation detection system 260 detects a cornea shape of the subject eye, for example. The deformation detection system 260 detects deformation of the cornea of the subject eye, for example. The deformation detection system 260 includes a light receiving lens 261, a diaphragm 262, and a light receiving element 263, for example. For example, the deformation detection system 260 may detect the deformation of the cornea based on corneal reflection light received by the light receiving element 263. For example, the deformation detection system 260 may detect the deformation of the cornea by receiving light emitted from the index light source 251 and reflected by the cornea of the subject eye, in which the light is received by the light receiving element 263. For example, the corneal reflected light passes through the objective 210 along the optical axis O1, and is reflected by the beam splitter 202 and the beam splitter 203. The corneal reflected light passes through the light receiving lens 261 and the diaphragm 262 along an optical axis O4, and is received by the light receiving element 263.

For example, the deformation detection system 260 may detect a deformed state of the cornea based on a magnitude of the light receiving signal of the light receiving element 236. For example, the deformation detection system 260 may detect that the cornea is in an applanation state when a light receiving amount of the light receiving element 236 is maximized. In this case, for example, the deformation detection system 260 is set to maximize the light receiving amount when the cornea of the subject eye is in the applanation state.

The deformation detection system 260 may be an anterior ocular segment image pickup unit such as an OCT or a Scheimpproof camera. For example, the deformation detection system 260 may detect a deformation amount or a deformation speed of the cornea.

The corneal thickness measuring system 270 measures a corneal thickness of the subject eye, for example. The corneal thickness measuring system 270 may include a light source 271, a light projecting lens 272, a diaphragm 273, a light receiving lens 274, and a light receiving element 275, for example. Light from the light source 271 passes through the light projecting lens 272 and the diaphragm 273 along an optical axis O5, and is irradiated to the subject eye. Reflected light reflected by the subject eye is condensed by the light receiving lens 274 along an optical axis O6, and is received by the light receiving element 275.

The Z alignment detection system 280 detects an alignment state in the Z direction, for example. The Z alignment detection system 280 includes a light receiving element 281, for example. The Z alignment detection system 280 may detect the alignment state in the Z direction, for example, by detecting reflected light from the cornea. For example, the Z alignment detection system 280 may receive reflected light emitted from the light source 271 and reflected by the cornea of the subject eye. In this case, the Z alignment detection system 280 may receive a bright spot generated by the light from the light source 271 being reflected by the cornea of the subject eye, for example. In this way, the light source 271 may be used as a light source for Z alignment detection. For example, light from the light source 271 reflected by the cornea is reflected by the beam splitter 204 along the optical axis O6, and is received by the light receiving element 281.

<Detection Unit>

A detection unit 500 detects an output of the ultrasonic actuator 100, for example. The detection unit 500 is, for example, a sensor such as an ultrasonic sensor, a displacement sensor, or a pressure sensor. The ultrasonic sensor detects an ultrasonic wave generated from the ultrasonic actuator 100. The displacement sensor detects a displacement of the ultrasonic actuator 100. The displacement sensor may continuously detect the displacement to detect vibration occurring when the ultrasonic actuator 100 generates the ultrasonic wave.

As shown in FIG. 2, the detection unit 500 is disposed outside an irradiation path A of the ultrasonic wave. The irradiation path A is, for example, a region connecting a front surface F of the ultrasonic actuator 100 and an irradiation target Ti of the ultrasonic wave. The detection unit 500 is disposed, for example, on the lateral side or the rear side of the ultrasonic actuator 100. As in the present example, in the case where the detection unit 500 is arranged on the lateral side, it is easy to observe the subject eye in the observation system 220. In the case where an ultrasonic sensor is used as the detection unit 500, the detection unit 500 detects ultrasonic waves leaking from the lateral side or the rear side of the ultrasonic actuator 100. In the case where a displacement sensor is used as the detection unit 500, the detection unit 500 detects the displacement of the ultrasonic actuator 100 from the lateral side or the rear side of the ultrasonic actuator 100. The displacement sensor irradiates the ultrasonic actuator 100 with laser light, for example, and detects the displacement of the ultrasonic actuator 100 based on the reflected laser light. A detection signal detected by the detection unit 500 is sent to a control unit.

<Control Unit>

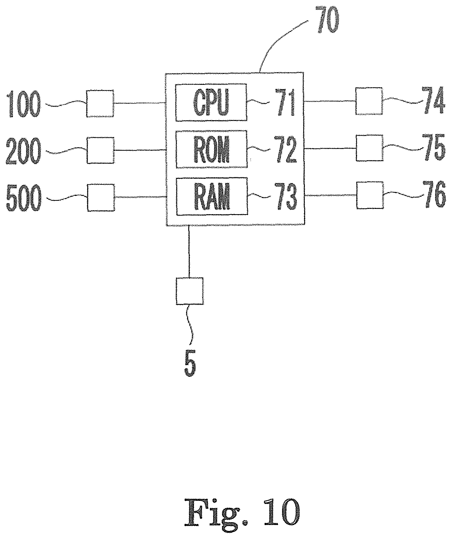

Next, the configuration of the control system will be described with reference to FIG. 10. A control unit 70 controls the entire apparatus and calculates a measurement value, for example. The control unit 70 is configured from a general central processing unit (CPU) 71, a ROM 72, and a RAM 73, for example. Various programs for controlling an operation of the ultrasonic tonometer 1 and initial values are stored in the ROM 72. The RAM 73 temporarily stores various information. The control unit 70 may be configured by one control unit or a plurality of control units (that is, a plurality of processors). The control unit 70 may be connected to, for example, the drive unit 5, a storage unit 74, a display unit 75, an operation unit 76, the ultrasonic actuator 100, the optical unit 200, and the detection unit 500.

The storage unit 74 is a non-transitory storage medium that can retain stored contents even though power supply is cut off. For example, a hard disk drive, a flash ROM, or a removable USB memory can be used as the storage unit 74.

The display unit 75 displays a measurement result of the subject eye, for example. The display unit 75 may include a touch panel function.

The operation unit 76 receives various operation instructions from an examiner. The operation unit 76 outputs an operation signal according to the input operation instruction to the control unit 70. The operation unit 76 may be, for example, a user interface of at least one of a touch panel, a mouse, a joystick, and a keyboard. In the case where the display unit 75 is a touch panel, the display unit 75 may function as the operation unit 76.

<Measurement Operation>

A control operation of the ultrasonic tonometer 1 having the above configuration will be described. At first, the control unit 70 performs alignment of the ultrasonic tonometer 1 with respect to a subject eye of a subject whose face is supported by the face support unit 4. For example, the control unit 70 detects a bright spot by the index projection system 250 from an anterior ocular front image acquired by the light receiving element 222, and drives the drive unit 5 so that a position of the bright spot becomes a predetermined position. Of course, the examiner may manually perform alignment on the subject eye using the operation unit 76 or the like while viewing the display unit 75. When the control unit 70 drives the drive unit 5, the control unit 70 determines whether the alignment is appropriate based on whether the position of the bright spot of the anterior ocular image is the predetermined position or not.

After completing the alignment on the subject eye E, the control unit 70 measures the corneal thickness by the corneal thickness measuring system 270. For example, the control unit 70 calculates the corneal thickness based on the light receiving signal received by the light receiving element 275. For example, the controller 70 may obtain the corneal thickness from a positional relationship between a peak value of reflected light on the front surface of the corneal and a peak value of reflected light on the back surface of the cornea, based on the received light signal. The control unit 70 stores, for example, the calculated corneal thickness in the storage unit 74 or the like.

Subsequently, the control unit 70 measures the intraocular pressure of the subject eye using the ultrasonic actuator 100. For example, the control unit 70 applies a voltage to the ultrasonic element 110 and irradiates the subject eye E with the ultrasonic wave. For example, the control unit 70 deforms the cornea by generating an acoustic radiation pressure by the ultrasonic wave. Then, the control unit 70 detects the deformed state of the cornea by the deformation detection system 260. For example, the control unit 70 detects that the cornea is deformed into a predetermined shape (applanation state or flat state) based on the light receiving signal of the light receiving element 263.

For example, the control unit 70 calculates the intraocular pressure of the subject eye based on the acoustic radiation pressure when the cornea of the subject eye deforms into the predetermined shape. The acoustic radiation pressure applied to the subject eye correlates with an irradiation time of the ultrasonic wave, and increases as the irradiation time of the ultrasonic wave increases. Therefore, the control unit 70 obtains the acoustic radiation pressure at the moment that the cornea is deformed into the predetermined shape based on the irradiation time of the ultrasonic wave. The relationship between the acoustic radiation pressure at the moment that the cornea is deformed into the predetermined shape and the intraocular pressure of the subject eye is obtained in advance by experiments or the like, and the relationship is stored in the storage unit 74 or the like. The control unit 70 determines the intraocular pressure of the subject eye based on the acoustic radiation pressure at the moment that the cornea is deformed into the predetermined shape and the relationship stored in the storage unit 74.

The method of calculating the intraocular pressure is not limited to the above, and various methods may be used. For example, the control unit 70 may obtain the intraocular pressure by obtaining the deformation amount of the cornea by the deformation detection system 260 and multiplying the deformation amount by a conversion factor. For example, the control unit 70 may correct an intraocular pressure value calculated according to the corneal thickness stored in the storage unit 74.

The control unit 70 may measure the intraocular pressure based on the ultrasonic wave reflected by the subject eye. For example, the control unit 70 may measure the intraocular pressure based on a change in the characteristics of the ultrasonic wave reflected by the subject eye, or may acquire the deformation amount of the cornea from the ultrasonic wave reflected by the subject eye and measure the intraocular pressure based on the deformation amount.

[Description of Reference Numerals]

1 ultrasonic tonometer
2 base
3 housing
4 face support unit
5 drive unit
70 control unit
100 ultrasonic actuator
110 ultrasonic element
131 sonotrode
132 back mass
180 uneven portion
181 thick portion
182 thin portion
183 curvature portion
184 irradiation surface
185 inner groove
186 outer groove
200 optical unit
400 mount unit
500 detection unit

## Claims

1. An ultrasonic actuator (100) that emits an ultrasonic wave, comprising:

    an ultrasonic element (110) generating an ultrasonic wave; and
    a sonotrode (131) propagating the ultrasonic wave generated from the ultrasonic element (110),
    wherein the sonotrode (131) includes:

        an irradiation surface (184) arranged between an outer surface and an inner surface of the sonotrode (131) and outputting the ultrasonic wave;
        an opening (101), defining the inner surface of the sonotrode, wherein the irradiation surface (184) is inclined to a side of the ultrasonic element (110) and toward a center of the opening (101); **characterized in that** the sonotrode comprises by

a groove (185) having a curved surface and formed inside the opening (101), which reduces a difference of a creepage distance between the outer surface and the inner surface of the sonotrode (131) to have a substantially same creepage distance until the ultrasonic wave generated from the ultrasonic element (110) reaches the irradiation surface (184),

wherein the creepage distance is a propagation distance of the ultrasonic wave in a direction of a sound axis (Q1) along the outer surface or the inner surface of the sonotrode (131).

2. The ultrasonic actuator (100) according to claim 1, wherein the ultrasonic actuator (100) is Langevin type.

3. An ultrasonic tonometer (1) for measuring an intraocular pressure of a subject's eye using an ultrasonic wave, comprising the actuator (100) of claim 1 or 2.

## Patentansprüche

1. Ultraschalllaktuator (100), der eine Ultraschallwelle emittiert, umfassend:

    ein Ultraschallelement (110), das eine Ultraschallwelle generiert; und
    eine Sonotrode (131), die die von dem Ultraschallelement (110) generierte Ultraschallwelle ausbreitet,
    wobei die Sonotrode (131) umfasst:

        eine Irradiationsoberfläche (184), die zwischen einer äußeren Oberfläche und einer inneren Oberfläche der Sonotrode (131) angeordnet ist und die Ultraschallwelle ausgibt;
        eine Öffnung (101), die die innere Oberfläche der Sonotrode definiert,
        wobei die Irradiationsoberfläche (184) zu einer Seite des Ultraschallelements (110) und in Richtung eines Zentrums der Öffnung (101) geneigt ist;
        **dadurch gekennzeichnet, dass** die Sonotrode umfasst
        eine Nut (185), die eine gekrümmte Oberfläche aufweist und innerhalb der Öffnung (101) geformt ist, die eine Differenz einer Kriechstrecke zwischen der äußeren Oberfläche und der inneren Oberfläche der Sonotrode (131) verringert, sodass sich im Wesentlichen dieselbe Kriechstrecke ergibt, bis die von dem Ultraschallelement (110) erzeugte Ultraschallwelle die Irradiationsoberfläche (184) erreicht,

wobei die Kriechstrecke eine Ausbreitungsstrecke der Ultraschallwelle in einer Richtung einer Schallachse (Q1) entlang der äußeren Oberfläche oder der inneren Oberfläche der Sonotrode (131) ist.

**2.** Ultraschallaktuator (1) nach Anspruch 1,
wobei der Ultraschallaktuator (100) vom Langevin-Typ ist.

**3.** Ultraschalltonometer (1) zum Messen eines intraokularen Drucks eines Objektauges unter Verwendung einer Ultraschallwelle, umfassend den Aktuator (100) nach Anspruch 1 oder 2.

**Revendications**

**1.** Actionneur ultrasonore (100) qui émet une onde ultrasonore, comprenant :

un élément ultrasonore (110) générant une onde ultrasonore ; et
une sonotrode (131) propageant l'onde ultrasonore générée par l'élément ultrasonore (110),
dans lequel la sonotrode (131) comprend :

une surface d'irradiation (184) agencée entre une surface extérieure et une surface intérieure de la sonotrode (131) et délivrant en sortie l'onde ultrasonore ;
une ouverture (101), définissant la surface intérieure de la sonotrode, dans lequel la surface d'irradiation (184) est inclinée vers un côté de l'élément ultrasonore (110) et vers un centre de l'ouverture (101) ; **caractérisé en ce que** la sonotrode comprend une rainure (185) ayant une surface incurvée et formée à l'intérieur de l'ouverture (101), qui réduit une différence de distance de glissement entre la surface extérieure et la surface intérieure de la sonotrode (131) pour avoir une distance de glissement sensiblement identique jusqu'à ce que l'onde ultrasonore générée par l'élément ultrasonore (110) atteigne la surface d'irradiation (184),
dans lequel la distance de glissement est une distance de propagation de l'onde ultrasonore dans une direction d'un axe sonore (Q1) le long de la surface extérieure ou de la surface intérieure de la sonotrode (131).

**2.** Actionneur ultrasonore (100) selon la revendication 1, dans lequel l'actionneur ultrasonore (100) est de type Langevin.

**3.** Tonomètre ultrasonore (1) pour mesurer une pression intraoculaire de l'œil d'un sujet à l'aide d'une onde ultrasonore, comprenant l'actionneur (100) de la revendication 1 ou 2.

Fig. 1

Fig. 2

EP 3 856 040 B1

Fig. 3

Fig. 4

φa

M 1

φb

Fig. 5A

φc

M 2

φd

Fig. 5B

Fig. 6

Fig. 7A

Fig. 7B

Fig. 8

Fig. 9

Fig. 10

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 2459268 A1
- JP H05253190 A

- JP 2009268651 A